# EUROPEAN PATENT APPLICATION

(11) **EP 4 574 881 A1**
(43) Date of publication of application: **25.06.2025**
(21) Application number: 23855079.2
(22) Date of filing: 02.08.2023
(51) Int. Cl.: C08G 63/60, C08G 63/06, C08G 63/183, C08G 63/199, C08J 11/24, C07C 69/82, C07C 69/017

(54) **POLYESTER RESIN COMPRISING BIS(GLYCOL) TEREPHTHALATE, AND PREPARATION METHOD THEREFOR**

(30) Priority: 17.08.2022 KR 20220102488
(71) Applicant: SK Chemicals Co., Ltd., Seongnam-si, Gyeonggi-do 13494 (KR)
(72) Inventor: HWANG, Da-Young, Seongnam-si, Gyeonggi-do 13494 (KR); LEE, Yoo Jin, Seongnam-si, Gyeonggi-do 13494 (KR)
(74) Representative: Haseltine Lake Kempner LLP
(86) International application number: PCT/KR2023/011291
(87) International publication number: WO 2024/039112

(57) **Abstract**

Bis(glycol) terephthalate is prepared by transesterification of bis(2-hydroxyethyl) terephthalate, which is a recycled monomer obtained by depolymerization of a waste polyester, and a glycol having at least 3 carbon atoms, and then is copolymerized, and thus can increase the amount of recycled monomers in a polyester resin, and has improved purity through the removal of impurities during transesterification, thereby enhancing final resin quality such as color.

## Description

### Technical Field

The present invention relates to a polyester resin comprising a bis(glycol) terephthalate as a recycled monomer and to a process for preparing the same.

### Background Art

Polyester is widely used as a material for beverage-filling containers, packaging films, audio and video films, and the like by virtue of its excellent mechanical strength, thermal resistance, transparency, and gas barrier properties. In addition, polyester is widely produced worldwide as an industrial material such as medical fibers and tire cords. In particular, polyester sheets or plates have good transparency and excellent mechanical strength, so that they are widely used as raw materials for cases, boxes, partitions, shelves, panels, packaging materials, building materials, interior and exterior materials, and the like.

As a result, waste of plastics such as polyester is generated globally at an unmanageable level every year. Recently, countries around the world have prepared regulations and plans for recycling waste plastic resources, including waste polyester. For example, there is an attempt to use a recycled resin in packaging materials used in various fields at a certain ratio or more. Although physical or chemical methods are used as methods of recycling waste polyester, physical recycling methods cannot guarantee purity and are not widely used.

In chemical recycling methods, the ester bond of waste polyester is cleaved to depolymerize it. Reactions such as glycolysis, hydrolysis, methanolysis, and aminolysis are used. Glycolysis among them is to decompose waste polyester by adding a glycol such as ethylene glycol or diethylene glycol at high temperatures. A reaction product containing mainly bis(2-hydroxyethyl) terephthalate (BHET) is obtained. The bis(2-hydroxyethyl) terephthalate may be used as a raw material for preparing unsaturated polyester or ester polyol after the crystallization or purification thereof.

In this regard, Korean Patent No. 1386683 discloses a crystallization method and apparatus for the chemical recycling of waste polyester. U.S. Patent No. 7,211,193 discloses a method for purifying bis(2-hydroxyethyl) terephthalate (BHET), which comprises subjecting a solution produced by the decomposition of a polyester containing polyethylene terephthalate (PET) as the main component using ethylene glycol (EG) to crystallization under a certain temperature condition and to solid-liquid separation.

### [Prior Art Document]

(Patent Document 1) Korean Patent No. 1386683
(Patent Document 2) U.S. Patent No. 7211193

### Disclosure of the Invention

### Technical Problem

The present inventors have attempted to develop a method capable of enhancing the quality of a final product while increasing the content of recycled monomers through a technology to recycle BHET obtained by the depolymerization of waste PET-based products into various engineering polyester products or environmentally friendly biodegradable polyester products.

Meanwhile, recycled BHET has generally low purity due to impurities formed from the reagents used in the depolymerization process and side reactions, so that separate processes such as ion exchange or recrystallization are required to purify it, thereby causing a problem of increased costs. In addition, glycol monomers (DEG, ISB, CHDM, and the like) must be used in addition to BHET in order to produce a copolyester resin, making it difficult to increase the content of recycled monomers in the final resin.

As a result of research conducted by the present inventors, it has been discovered that, when a glycol for copolymerization is subjected to a transesterification reaction with BHET in advance to prepare a bis(glycol) terephthalate, which is then subjected to a polymerization reaction, the content of recycled monomers in the polyester resin can be increased; and purity is improved by removing impurities during the transesterification reaction, thereby enhancing the quality, such as color, of the final resin.

Accordingly, an object of the present invention is to provide a process for preparing a polyester resin with a high content of recycled monomers such as bis(2-hydroxyethyl) terephthalate and excellent quality of the final resin, and a polyester resin prepared thereby.

### Solution to the Problem

According to the present invention, there is provided a process for preparing a polyester resin, which comprises subjecting bis(2-hydroxyethyl) terephthalate to a reaction with a glycol having 3 or more carbon atoms to prepare a bis(glycol) terephthalate; and preparing a copolymer using the bis(glycol) terephthalate and bis(2-hydroxyethyl) terephthalate.

According to the present invention, there is provided a polyester resin, which comprises bis(2-hydroxyethyl) terephthalate and a bis(glycol) terephthalate as comonomers, wherein the glycol has 3 or more carbon atoms.

### Advantageous Effects of the Invention

According to the present invention, as bis(2-hydroxyethyl) terephthalate obtained by the depolymerization of waste polyester is subjected to a transesterification reaction with a glycol having 3 or more carbon atoms to prepare a bis(glycol) terephthalate, which is then subjected to copolymerization, the content of recycled monomers in the polyester resin can be increased, and purity is improved by removing impurities during the transesterification reaction, thereby enhancing the quality, such as color, of the final resin.

In addition, according to the present invention, as a glycol monomer suitable for the desired copolymerization composition of a polyester resin is subjected to a transesterification reaction with bis(2-hydroxyethyl) terephthalate in advance, glycol by-products formed in the polymerization reaction can be reduced, and the purity of the recycled monomer is improved even when bis(2-hydroxyethyl) terephthalate with low purity is used, thereby reducing the costs for raw materials and processing. Thus, it can be applied to the preparation of environmentally friendly polyester articles.

### Best Mode for Carrying out the Invention

Hereinafter, the present invention will be described in more detail.

In this specification, terms referring to the respective components are used to distinguish them from each other and are not intended to limit the scope of the embodiment. In addition, in the present specification, a singular expression is interpreted to cover a plural number as well unless otherwise specified in the context.

In the present specification, the terms first, second, and the like are used to describe various components. But the components should not be limited by the terms. The terms are used for the purpose of distinguishing one element from another.

In the present specification, the term "comprising" is intended to specify a particular characteristic, region, step, process, element, and/or component. It does not exclude the presence or addition of any other characteristic, region, step, process, element, and/or component, unless specifically stated to the contrary.

The molecular weight of a compound or polymer described in the present specification, for example, a number average molecular weight or a weight average molecular weight, is a relative mass based on carbon-12 as is well known. Although its unit is not described, it may be understood as a molar mass (g/mole) of the same numerical value, if necessary.

In the present specification, a "derivative" of a specific compound refers to a compound obtained by partially transforming the compound through a chemical reaction or combining it with other components, thereby containing the main part of the compound.

In the present specification, a unit or group "derived" from a specific component refers to a part of the component contained in a final product through a chemical reaction such as a polymerization reaction. It may be present in a form in which a part thereof is modified during the reaction or it is combined with other components. For example, a unit or group derived from at least one monomer is contained in the chain constituting a polymer.

In the present specification, a "recycled monomer" may refer to a monomer obtained by decomposing, depolymerizing, reprocessing, or repolymerizing waste plastics such as waste polyester by physical or chemical methods, or polymerization raw materials containing the monomer or derived from the monomer.

As an example, bis(2-hydroxyethyl) terephthalate (BHET) used as a starting material in the process according to the present invention may be a recycled monomer obtained by the depolymerization of waste polyester, and a bis(glycol) terephthalate (BHDT, BHIT, and BHCT) prepared therefrom may also be considered a recycled monomer.

As another example, recycled monomers may also be used as a glycol having 3 or more carbon atoms to be subjected to a transesterification reaction in the process of the present invention. As a specific example, at least one type of recycled glycol, such as recycled 1,4-cyclohexanedimethanol (r-CHDM), recycled diethylene glycol (r-DEG), and recycled isosorbide (r-ISB) may be used.

As another example, recycled monomers may also be used as a comonomer to be subjected to a polymerization reaction for a polyester resin in the process of the present invention. As a specific example, at least one type selected from recycled dicarboxylic acids and recycled diols may be used. As a more specific example, at least one type of recycled monomer, such as recycled terephthalic acid (r-TPA), recycled isophthalic acid (r-IPA), recycled dimethyl terephthalate (r-DMT), recycled ethylene glycol (r-EG), recycled 1,4-cyclohexanedimethanol (r-CHDM), recycled diethylene glycol (r-DEG), and recycled isosorbide (r-ISB) may be used.

Such recycled monomers may be obtained directly from waste plastics such as waste polyester by known methods or can be purchased commercially and used.

According to an aspect of the present invention, there is provided a process for preparing a polyester resin, which comprises subjecting bis(2-hydroxyethyl) terephthalate to a reaction with a glycol having 3 or more carbon atoms to prepare a bis(glycol) terephthalate; and preparing a copolymer using the bis(glycol) terephthalate and bis(2-hydroxyethyl) terephthalate.

The process for preparing a bis(glycol) terephthalate comprises feeding a glycol having 3 or more carbon atoms to a reactor; and feeding bis(2-hydroxyethyl) terephthalate to the reactor and carrying out a transesterification reaction.

The process of the present invention provides a polyester resin with a high content of recycled monomers such as bis(2-hydroxyethyl) terephthalate and excellent quality of the final resin. Specifically, as a glycol monomer suitable for the desired copolymerization composition of a polyester resin is subjected to a transesterification reaction with bis(2-hydroxyethyl) terephthalate in advance, glycol by-products formed in the polymerization reaction can be reduced, and the purity of the recycled monomer is improved even when bis(2-hydroxyethyl) terephthalate with low purity is used, thereby reducing the costs for raw materials and processing. Thus, it can be applied to the preparation of environmentally friendly polyester articles.

### Bis(2-hydroxyethyl) terephthalate

Bis(2-hydroxyethyl) terephthalate is an ester of two ethylene glycols and one terephthalic acid. For example, it is a compound formed as an intermediate in the process of preparing a polyester such as polyethylene terephthalate (PET) through the polymerization of ethylene glycol and terephthalic acid or its ester.

The bis(2-hydroxyethyl) terephthalate used in the present invention may be obtained by the depolymerization of waste polyester. For example, the bis(2-hydroxyethyl) terephthalate may be obtained from waste polyester having a repeat unit of ethylene glycol and terephthalic acid like polyethylene terephthalate (PET) or glycol-modified polyethylene terephthalate (PETG). Specifically, it may be obtained by well-known depolymerization methods such as glycolysis, hydrolysis, and methanolysis. In particular, the bis(2-hydroxyethyl) terephthalate may be obtained by depolymerizing waste polyethylene terephthalate with ethylene glycol and then purifying it.

Bis(2-hydroxyethyl) terephthalate obtained by the depolymerization of waste polyester as described above is referred to as "recycled bis(2-hydroxyethyl) terephthalate (recycled BHET)," or abbreviated as r-BHET or rBHET, which needs to be understood as distinct from a pure BHET compound. Specifically, recycled BHET may contain reagents or solvents used in various chemical steps during the depolymerization of waste polyester or by-products formed by side reactions with them. Thus, BHET recycled by a common depolymerization process contains organic and inorganic impurities in addition to BHET as the main component; thus, its purity is not high. For this reason, recycled BHET can also be viewed as a kind of composition comprising two or more components, i.e., a BHET composition. Such recycled BHET may be used as a polymerization raw material for producing a polyester resin.

Impurities contained in the recycled BHET may comprise, for example, diethylene glycol derivatives and unreacted monomers. The total content of impurities contained in the recycled BHET may be 10% by weight or more, 15% by weight or more, or 20% by weight or more, and may be 40% by weight or less, 35% by weight or less, 30% by weight or less, or 25% by weight or less.

The purity of the recycled BHET may be measured using liquid chromatography or the like. Specifically, the purity of the recycled BHET may be calculated by measuring the fraction (%) of the peak area of BHET out of the total peak area in a spectrum obtained using high-performance liquid chromatography (HPLC).

For example, the purity of the recycled BHET may be 97% or less, 90% or less, 85% or less, or 80% or less, and may be 60% or more, 65% or more, or 70% or more. Specifically, the purity of the BHET introduced into the transesterification reaction of the present invention may be 60% to 97%, more specifically, 65% to 90%, 65% to 85%, or 70% to 80%.

### Glycol

The glycol used in the transesterification reaction of the present invention is subjected to a transesterification reaction with bis(2-hydroxyethyl) terephthalate to form a residue in a bis(glycol) terephthalate as a product. It constitutes the polymer chain of a final polyester resin polymerized from the bis(glycol) terephthalate.

The glycol used in the present invention may be a glycol having 3 or more carbon atoms for substituting an ethylene glycol residue in a transesterification reaction.

In particular, the glycol has a boiling point higher than that of ethylene glycol by 10°C or more, which is advantageous for purification through fractional distillation during the transesterification reaction.

Specifically, the glycol used in the transesterification reaction of the present invention may be a glycol monomer other than ethylene glycol (e.g., alkylene glycol having 3 or more carbon atoms) or a polymer glycol (e.g., polyether).

As a more specific example, the glycol may be at least one selected from glycol monomers having 3 to 20 carbon atoms and having a molecular weight of less than 500 and polymeric glycols having a number average molecular weight of 400 to 5,000.

The number of carbon atoms of the glycol monomer may be, for example, 3 or more or 4 or more, and may be 20 or less, 15 or less, 12 or less, 10 or less, or 8 or less.

Specifically, the glycol monomer may be an aliphatic diol having 3 to 20 carbon atoms. In addition, the aliphatic diol may be a chain type or a cyclic type.

As a specific example, the glycol having 3 or more carbon atoms may comprise at least one selected from the group consisting of 1,3-propanediol, 1,4-butanediol, 1,4-cyclohexanedimethanol, isosorbide, 2-methyl-1,3-propanediol, 2-methylene-1,3-propanediol, 2-ethyl-1,3-propanediol, 2-isopropyl-1,3-propanediol, 2,2-dimethyl-1,3-propanediol, 2,3-butanediol, 3-methyl-1,5-pentanediol, 3-methyl-2,4-pentanediol, 1,6-hexanediol, 1,2-cyclohexanediol, 1,4-cyclohexanediol, diethylene glycol, polyethylene glycol, polypropylene glycol, polytetramethylene glycol, polyhexamethylene glycol, copolymers of ethylene oxide and tetrahydrofuran, ethylene oxide-adducted polypropylene glycol, polycarbonate diol, polyneopentyl glycol, poly-3-methylpentanediol, poly-1,5-pentanediol, and derivatives thereof.

As a more specific example, the glycol monomer may comprise at least one selected from the group consisting of diethylene glycol, isosorbide, 1,4-cyclohexanedimethanol, and derivatives thereof. Such a glycol monomer may be a virgin glycol.

As another example, the glycol having 3 or more carbon atoms may comprise at least one type of recycled glycol obtained by the depolymerization of waste polyester. As a specific example, the recycled glycol may be selected from the group consisting of recycled 1,4-cyclohexanedimethanol, recycled diethylene glycol, and recycled isosorbide. However, the type of recycled glycol that can be used in the present invention is not limited thereto. Any recycled glycol that can be subjected to a transesterification reaction can be used. Such a recycled glycol may be used in the transesterification reaction to completely replace a virgin glycol. Alternatively, a recycled glycol and a virgin glycol may be mixed at a certain ratio and used in the transesterification reaction. The mixing ratio of the recycled glycol may be, for example, 0% by mole or more, 1% by mole or more, 10% by mole or more, 20% by mole or more, 30% by mole or more, 50% by mole or more, or 60% by mole or more, and 100% by mole or less, 99% by mole or less, 90% by mole or less, 50% by mole or less, or 40% by mole or less, specifically, 1% by mole to 100% by mole, 1% by mole to 99% by mole, or 30% by mole to 100% by mole, based on the number of moles of the total glycols.

Specific examples of the derivative of 1,4-cyclohexanedimethanol may include 4-(hydroxymethyl)cyclohexylmethyl-4-(hydroxymethyl)cyclohexanecarboxylate, 4-(4-(hydroxymethyl)cyclohexylmethoxymethyl)cyclohexylmethanol, or a mixture thereof. More specifically, it may be a mixture comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:1 to 1:5 or 1:2 to 1:4.

The glycol monomer may have a molecular weight of, for example, less than 500, less than 400, less than 350, less than 300, or less than 250.

The polymer glycol may be, for example, selected from the group consisting of polyethylene glycol, polypropylene glycol, polytetramethylene glycol, polyhexamethylene glycol, copolymers of ethylene oxide and tetrahydrofuran, ethylene oxide-adducted polypropylene glycol, polycarbonatediol, polyneopentyl glycol, poly-3-methylpentanediol, and poly-1,5-pentanediol. More specifically, the polymer glycol may be at least one selected from the group consisting of polytetramethylene glycol, polycarbonatediol, polypropylene glycol, and ethylene oxide-adducted polypropylene glycol.

The polymer glycol may have a number average molecular weight of, for example, 400 or more, 500 or more, 600 or more, 700 or more, or 800 or more, and may be 6,000 or less, 5,000 or less, 4,000 or less, or 3,000 or less. As a specific example, the number average molecular weight of the polymer glycol may be 400 to 5,000. More specifically, it may preferably be 1,000 to 3,000 from the viewpoint of the reduction in phase separation.

The polymer glycol is used in an amount of 5% by weight to 75% by weight, specifically, 10% by weight to 60% by weight, more specifically, 15% by weight to 50% by weight, based on the weight of a final polyester resin, which is advantageous for achieving a high molecular weight while improving the elasticity of the polyester resin.

### Transesterification reaction

The glycol and bis(2-hydroxyethyl) terephthalate are subjected to a transesterification reaction.

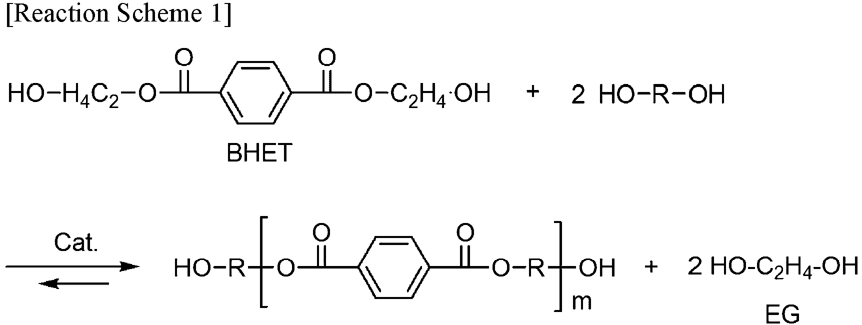

In Reaction Scheme 1, m is, for example, a number in the range of 1 to 10 or 1 to 4.

In addition, in Reaction Scheme 1, R is a group of a glycol having 3 or more carbon atoms from which the OH groups at both ends are excluded. As an example, R may be an alkylene group having 3 to 20 carbon atoms or a group in which two or more identical or different alkylene groups having 2 to 10 carbon atoms are connected via an ether group or a carbonate group. As another example, R may be a group of 3 to 20 carbon atoms containing single or multiple rings, wherein the ring may be aliphatic or aromatic and may also contain one or more heteroatoms (e.g., O, N, and S).

The transesterification reaction may be carried out in the presence of a catalyst. Thus, when the glycol, acid, or bis(2-hydroxyethyl) terephthalate is fed to a reactor, a catalyst may be fed together.

As the catalyst for the transesterification reaction, for example, at least one selected from the group consisting of a zinc-based catalyst, a titanium-based catalyst, a germanium-based catalyst, an antimony-based catalyst, an aluminum-based catalyst, and a tin-based catalyst may be used.

Examples of the zinc-based catalyst include zinc acetate, zinc acetate hydrate, zinc chloride, zinc sulfate, zinc sulfide, zinc carbonate, zinc citrate, zinc gluconate, or mixtures thereof. Examples of the titanium-based catalyst include tetraethyl titanate, acetyltripropyl titanate, tetrapropyl titanate, tetrabutyl titanate, 2-ethylhexyl titanate, octylene glycol titanate, triethanolamine titanate, acetylacetonate titanate, ethylacetoacetic ester titanate, isostearyl titanate, titanium dioxide, and the like. Examples of the germanium-based catalyst include germanium dioxide, germanium tetrachloride, germanium ethylene glycol oxide, germanium acetate, or combinations thereof. Specifically, germanium dioxide can be used as the germanium-based catalyst. Both crystalline and amorphous germanium dioxide may be used as the germanium dioxide, and glycol-soluble ones may also be used.

The amount of the transesterification catalyst employed may vary depending on the reaction conditions and the catalyst used. As an example, a metal-based catalyst (e.g., titanium-based catalyst, tin-based catalyst) may be employed in an amount of 0.0001 part by weight to 0.05 part by weight relative to 100 parts by weight of the glycol and bis(2-hydroxyethyl) terephthalate fed to a reactor.

The transesterification reaction may be carried out in a batch or continuous type.

As an example, when a glycol, or a glycol with an acid, is fed to a reactor, and the temperature reaches a certain level while the temperature is raised, bis(2-hydroxyethyl) terephthalate may be fed. The feeding of bis(2-hydroxyethyl) terephthalate may be carried out, for example, in a nitrogen atmosphere at a temperature of 180°C to 280°C while ethylene glycol as a by-product is removed.

The bis(2-hydroxyethyl) terephthalate may be fed all at once to be subjected to a transesterification reaction with the glycol. As a specific example, bis(2-hydroxyethyl) terephthalate may be prepared in a powder form or in an aqueous BHET solution having a concentration of about 10 to 20% by weight as dissolved in water at about 80 to 100°C, which may be fed to a reactor all at once.

Alternatively, bis(2-hydroxyethyl) terephthalate may be introduced into the transesterification reaction with a glycol in a divided or continuous manner. According to an embodiment, bis(2-hydroxyethyl) terephthalate may be introduced into the transesterification reaction with a glycol in a divided manner of two or more times. The number of divided introductions may be 2 times or more, 3 times or more, 4 times or more, or 5 times or more, and may be 100 times or fewer, 50 times or fewer, 30 times or fewer, 20 times or fewer, 15 times or fewer, or 10 times or fewer. As a specific example, the number of divided introductions may be 2 to 30 times or 3 to 15 times. The time interval between the divided introductions may be determined by dividing the total introduction time by the number of divided introductions. The total introduction time may be, for example, 1 hour or longer or 2 hours or longer, and may be 5 hours or shorter or 4 hours or shorter. In addition, the amount of introduction at one time during the divided introductions may be determined by dividing the total amount of bis(2-hydroxyethyl) terephthalate to be introduced into the reaction by the number of divided introductions.

According to another embodiment, bis(2-hydroxyethyl) terephthalate is introduced into the transesterification reaction with a glycol in a continuous manner. The total time of continuous introduction may be, for example, 1 hour or longer or 2 hours or longer, and may be 5 hours or shorter or 4 hours or shorter. For example, the continuous introduction may be to introduce a constant amount of bis(2-hydroxyethyl) terephthalate per hour. The amount of introduction per hour may be determined by dividing the total amount of bis(2-hydroxyethyl) terephthalate to be introduced into the reaction by the total introduction time. As a specific example, bis(2-hydroxyethyl) terephthalate may be prepared in a powder form or in an aqueous BHET solution having a concentration of about 10 to 20% by weight as dissolved in water at about 80 to 100°C, which may be continuously fed. The continuous introduction may be carried out from the beginning of the transesterification reaction until one hour before the completion of the reaction. A dropping funnel may be used at the laboratory level, or a metered feeder may be used at the commercial level, for the continuous introduction of a constant amount per hour.

When the feeding of bis(2-hydroxyethyl) terephthalate is completed, the reaction conditions may be maintained until the transesterification reaction is completed. In addition, a process of collecting and removing ethylene glycol as a by-product may be continued during the transesterification reaction. The collection and removal of ethylene glycol may be carried out by a distillation process using a difference in boiling point from the other components. Ethylene glycol thus distilled off may be recovered by cooling and reused in other processes.

The termination point of the transesterification reaction may be determined by considering the theoretical amount of ethylene glycol formed from bis(2-hydroxyethyl) terephthalate through the transesterification reaction or as the point when the by-product is no longer released.

The pressure (absolute pressure) during the transesterification reaction may be, for example, 0.5 kgf/cm² or more, 0.7 kgf/cm² or more, or 1.0 kgf/cm² or more, and 2.5 kgf/cm² or less, 2.0 kgf/cm² or less, or 1.5 kgf/cm² or less, specifically, 0.5 kgf/cm² to 2.5 kgf/cm².

The temperature during the transesterification reaction may be 140°C or higher, 160°C or higher, 180°C or higher, or 200°C or higher, and may be 300°C or lower, 280°C or lower, 270°C or lower, 250°C or lower, or 220°C or lower. In addition, the transesterification reaction may be carried out under a nitrogen atmosphere. As a specific example, the transesterification reaction may be carried out at a pressure of 0.5 kgf/cm² to 2.5 kgf/cm² and a temperature of 180°C to 280°C under a nitrogen atmosphere.

The pressure and temperature conditions during the transesterification reaction may be appropriately adjusted according to the specific characteristics of a polyester to be produced, the ratio of each component, or process conditions. For example, the transesterification reaction may be carried out at a temperature of 180°C or higher to smoothly remove ethylene glycol, a by-product formed during the transesterification reaction. In addition, it may be carried out at a temperature lower than the boiling point of the glycol by 10°C in order to reduce the loss of the glycol for the substitution. As a specific example, when 1,4-butanediol is used as the glycol, the temperature may be adjusted to 180°C to 220°C. Alternatively, when 1,4-cyclohexanedimethanol is used, the temperature may be adjusted to 200°C to 270°C.

The product of the transesterification reaction mainly comprises a bis(glycol) terephthalate and derivatives thereof in which ethylene glycol residues in bis(2-hydroxyethyl) terephthalate are substituted with other glycol residues.

According to an embodiment, the bis(glycol) terephthalate obtained by the transesterification reaction comprises a compound represented by the following Formula 1.

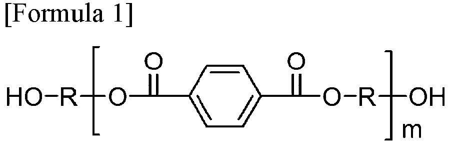

In Formula 1, -O-R-OH is a group derived from a glycol having 3 or more carbon atoms, and m is, for example, a number in the range of 1 to 10 or 1 to 4.

The compound of Formula 1 may be a monomer or oligomer (dimer, trimer, or the like) of a bis(glycol) terephthalate.

In Formula 1, R is a group derived from a glycol having 3 or more carbon atoms. As an example, R may be an alkylene group having 3 to 20 carbon atoms or a group in which two or more identical or different alkylene groups having 2 to 10 carbon atoms are connected via an ether group or a carbonate group. As another example, R may be a group of 3 to 20 carbon atoms containing single or multiple rings, wherein the ring may be aliphatic or aromatic and may also contain one or more heteroatoms (e.g., O, N, and S).

As a specific example, Formula 1 may be a compound in which R is a group derived from diethylene glycol and m is 1, that is, bis(diethylene glycol) terephthalate (hereinafter, abbreviated as BHDT). As another specific example, Formula 1 may be a compound in which R is a group derived from isosorbide and m is 1, that is, bis(isosorbide) terephthalate (hereinafter, abbreviated as BHIT). As another specific example, Formula 1 may be a compound in which R is a group derived from 1,4-cyclohexanedimethanol and m is 1, that is, bis(1,4-cyclohexanedimethanol) terephthalate (hereinafter, abbreviated as BHCT).

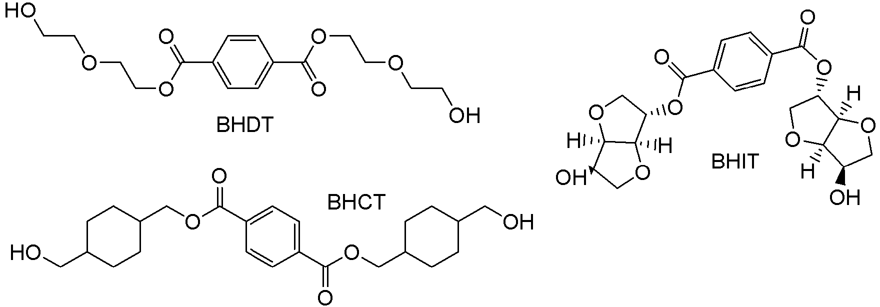

The bis(glycol)terephthalate may comprise two or more compounds represented by Formula 1 or derivatives thereof.

Since impurities in the bis(glycol) terephthalate are removed in the process of removing by-products such as a glycol formed during the transesterification reaction, its purity may be enhanced as compared with bis(2-hydroxyethyl) terephthalate as a starting material. For example, the bis(glycol) terephthalate obtained through the transesterification reaction may have a purity of 80% or more. More specifically, the purity of the bis(glycol) terephthalate may be 85% or more, 90% or more, or 95% or more. As a specific example, it may be 80% to 99.9%.

### Preparation of a polyester resin

Thereafter, a polyester resin is prepared through a step of preparing a copolymer using the bis(glycol) terephthalate and bis(2-hydroxyethyl) terephthalate.

In the step of preparing a copolymer, an esterification reaction (first polymerization reaction step) and a polycondensation reaction (second polymerization reaction step) may be sequentially carried out.

For example, the step of preparing a copolymer may comprise subjecting comonomers comprising the bis(glycol) terephthalate and bis(2-hydroxyethyl) terephthalate to an esterification reaction to obtain an oligomer; and subjecting the oligomer to a polycondensation to obtain a copolymer.

Reaction Scheme 2 below schematically shows the copolymerization reaction of a polyester during which a glycol may be formed as a by-product.

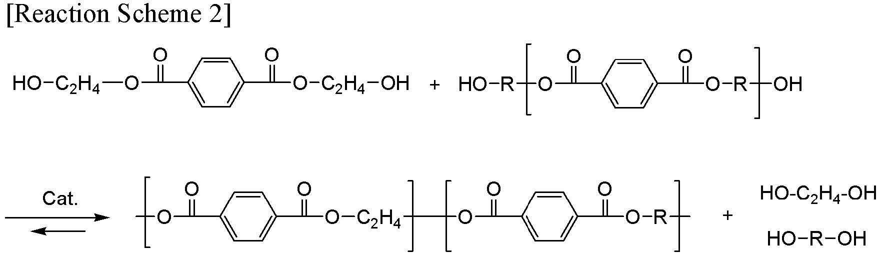

In Reaction Scheme 2, R is a group derived from a glycol having 3 or more carbon atoms.

Additional monomers in addition to the recycled monomer may be introduced into the esterification reaction. For example, at least one comonomer selected from the group consisting of a dicarboxylic acid, a dicarboxylic acid derivative, a diol, and a diol derivative may be further introduced into the esterification reaction.

As a specific example, the dicarboxylic acid may comprise terephthalic acid or isophthalic acid; the dicarboxylic acid derivative may comprise dimethyl terephthalate or dimethyl isophthalate; the glycol may comprise diethylene glycol, 1,4-cyclohexanedimethanol, isosorbide, 1,2-propanediol, 1,3-propanediol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 1,4-butanediol, 2,3-butanediol, 3-methyl-1,5-pentanediol, 3-methyl-2,4-pentanediol, 1,6-hexanediol, 1,2-cyclohexanediol, or 1,4-cyclohexanediol; and the diol derivative may comprise 4-(hydroxymethyl)cyclohexylmethyl-4-(hydroxymethyl)cyclohexanecarboxylate or 4-(4-(hydroxymethyl)cyclohexylmethoxymethyl)cyclohexylmethanol. Such comonomers may be virgin monomers.

As another example, the at least one comonomer may comprise a recycled monomer obtained by the depolymerization of waste polyester. As a specific example, the at least one comonomer may comprise at least one recycled monomer selected from the group consisting of a recycled dicarboxylic acid, a recycled dicarboxylic acid derivative, a recycled diol, and a recycled diol derivative. As a more specific example, the recycled monomer may be selected from the group consisting of recycled ethylene glycol, recycled 1,4-cyclohexanedimethanol, recycled diethylene glycol, recycled isosorbide, recycled terephthalic acid, recycled dimethyl terephthalate, recycled isophthalic acid, and recycled dimethyl isophthalate. However, the type of the recycled monomer that can be used in the present invention as a comonomer is not limited thereto. Any recycled monomer that can be used in the preparation of a polyester resin can be used. Such a recycled monomer may be used in the polymerization of a polyester resin by completely replacing a virgin monomer. Alternatively, a recycled monomer and a virgin monomer may be mixed at a certain ratio and used in the polymerization of a polyester resin. The mixing ratio of the recycled monomer may be, for example, 0% by mole or more, 1% by mole or more, 10% by mole or more, 20% by mole or more, 30% by mole or more, 50% by mole or more, or 60% by mole or more, and 100% by mole or less, 99% by mole or less, 90% by mole or less, 50% by mole or less, or 40% by mole or less, specifically, 1% by mole to 100% by mole, 1% by mole to 99% by mole, or 30% by mole to 100% by mole, based on the number of moles of the total monomers.

The esterification reaction may be carried out in the presence of an esterification reaction catalyst. For example, a zinc-based compound may be used. Specific examples of the zinc-based catalyst include zinc acetate, zinc acetate hydrate, zinc chloride, zinc sulfate, zinc sulfide, zinc carbonate, zinc citrate, zinc gluconate, or mixtures thereof.

The esterification reaction may be carried out, for example, at a pressure of 0 kgf/cm² to 10.0 kgf/cm² and a temperature of 150°C to 300°C. The esterification reaction conditions may be appropriately adjusted according to the specific characteristics of the polyester to be produced, the ratio of each component, or process conditions. Specifically, the pressure in the esterification reaction may be 0 kg/cm² to 5.0 kg/cm², more specifically, 0.1 kg/cm² to 3.0 kg/cm². In addition, the temperature in the esterification reaction may be 200°C to 270°C, more specifically, 240°C to 260°C.

The esterification reaction may be carried out in a batch or continuous type. In addition, recycled BHET, a dicarboxylic acid, and a diol, which are raw materials, may be separately introduced into the reactor, or two or more raw materials may be introduced in a mixed state. They may be introduced in a solid, liquid, or slurry form. As an example, the dicarboxylic acid, the diol, and the recycled BHET may be added individually or in combination and may be mixed with a terephthalic acid oligomer prepared in advance. The terephthalic acid oligomer may be prepared by, for example, reacting terephthalic acid with a diol such as ethylene glycol, cyclohexanedimethanol, and isosorbide. As another example, they may be introduced in the form of a slurry in which a dicarboxylic acid and recycled BHET are mixed with a diol.

More specifically, a diol such as isosorbide, which is solid at room temperature, may be dissolved in water or ethylene glycol and then mixed with a dicarboxylic acid such as terephthalic acid to prepare a slurry. Alternatively, isosorbide may be melted at 60°C or higher, which is then mixed with a dicarboxylic acid such as terephthalic acid and other diols to prepare a slurry. In addition, water may be additionally added to the mixed slurry to help increase the fluidity of the slurry. In addition, in a continuous type, liquid raw materials (e.g., a solution of recycled BHET) may be continuously fed to a reactor using a pump or the like. The hourly feeding amount of raw materials can be determined by dividing the total amount of raw materials to be fed by time to achieve the target production per day.

The mixture of the bis(2-hydroxyethyl) terephthalate, bis(glycol) terephthalate, and other additive components is left in the esterification reactor for a certain period of time, for example, 1 hour to 24 hours or 4 hours to 10 hours, which is then transferred to a polycondensation reactor. The polycondensation reaction may produce a polyester resin having a relatively low molecular weight through melt polymerization. In addition, a polyester resin having a relatively high molecular weight may be produced through solid-state polymerization after the melt polymerization.

The temperature in the polycondensation reaction may be 150°C to 300°C, specifically, 200°C to 290°C, more specifically, 260°C to 280°C. In addition, the pressure in the polycondensation reaction may be 0.01 mmHg to 600 mmHg, specifically, 0.05 mmHg to 200 mmHg, more specifically, 0.1 mmHg to 100 mmHg. As the reduced pressure condition is adopted in the polycondensation reaction, a glycol, which is a by-product of the polycondensation reaction, can be collected and removed from the system. If the pressure in the polycondensation reaction exceeds the range of 0.01 mmHg to 400 mmHg, the removal of by-products may be insufficient. In addition, if the temperature in the polycondensation reaction is lower than 150°C, a glycol as a by-product of the reaction cannot be effectively collected and removed from the system; thus, the intrinsic viscosity of a final reaction product is low, resulting in a decrease in the physical properties of the final polyester resin. If the temperature in the polycondensation reaction exceeds 300°C, the possibility of yellowing of a final polyester resin increases. In addition, the polycondensation reaction may be carried out for a necessary period of time, for example, an average residence time of 1 hour to 24 hours, until the intrinsic viscosity of a final reaction product reaches an appropriate level.

In addition, the polycondensation reaction may be carried out in the presence of a polycondensation catalyst. The polycondensation catalyst may be, for example, a titanium-based compound, a germanium-based compound, an antimony-based compound, an aluminum-based compound, a tin-based compound, or a mixture thereof. Examples of the titanium-based compound include tetraethyl titanate, acetyltripropyl titanate, tetrapropyl titanate, tetrabutyl titanate, 2-ethylhexyl titanate, octylene glycol titanate, lactate titanate, triethanolamine titanate, acetylacetonate titanate, ethylacetoacetic ester titanate, isostearyl titanate, titanium dioxide, and the like. Examples of the germanium-based compound include germanium dioxide, germanium tetrachloride, germanium ethylene glycol oxide, germanium acetate, or mixtures thereof. Preferably, germanium dioxide can be used. Both crystalline and amorphous germanium dioxide may be used, and glycol-soluble ones may also be used. The amount of the polycondensation catalyst used may be such that the amount of titanium element relative to the weight of the polyester resin is about 1 ppm to 100 ppm, more preferably, about 1 ppm to 50 ppm.

In addition to the polycondensation catalyst, a stabilizer, a colorant, a crystallizing agent, an antioxidant, a branching agent, or the like may be further used. The timing of adding these additives is not particularly limited, and they may be added at any time during the preparation step of the polyester resin.

As the stabilizer, phosphorus-based compounds such as phosphoric acid, trimethyl phosphate, triethyl phosphate, and triethyl phosphonoacetate may be generally used. The amount thereof added may be such that 10 ppm to 200 ppm relative to the weight of the polyester resin based on the amount of elements. In addition, common colorants such as cobalt acetate and cobalt propionate may be exemplified as the colorant added to enhance the color of the polyester resin. The amount thereof added may be such that 10 ppm to 200 ppm relative to the weight of the polyester resin based on the amount of cobalt element. If necessary, anthraquinone-based compounds, perinone-based compounds, azo-based compounds, methine-based compounds, or the like may be used as an organic colorant. Commercially available toners such as Polysynthren Blue RLS from Clarient or Solvaperm Red BB from Clarient may be used. The amount of the organic compound colorant added may be adjusted to 0 to 50 ppm based on the weight of the polyester resin. A crystal nucleating agent, an ultraviolet absorber, a polyolefin resin, a polyamide resin, and the like may be exemplified as the crystallizing agent. Hindered phenol-based antioxidants, phosphite-based antioxidants, thioether-based antioxidants, or mixtures thereof may be exemplified as the antioxidant. Conventional branching agents having three or more functional groups, for example, trimellitic anhydride, trimethylol propane, trimellitic acid, or mixtures thereof may be exemplified as the branching agent.

### Polyester resin

According to another aspect of the present invention, there is provided a polyester resin prepared by the above process.

The polyester resin of the present invention is a polyester resin regenerated through the chemical recycling of waste polyester.

That is, the polyester resin is prepared by copolymerizing monomers comprising bis(2-hydroxyethyl) terephthalate and a bis(glycol) terephthalate. Accordingly, the polyester resin comprises bis(2-hydroxyethyl) terephthalate and a bis(glycol) terephthalate as a comonomer. Here, the glycol has 3 or more carbon atoms.

As described above, the bis(glycol) terephthalate may be a transesterification product between bis(2-hydroxyethyl) terephthalate and a glycol having 3 or more carbon atoms.

Here, the glycol has 3 or more carbon atoms, and the specific types thereof are as exemplified above.

As a specific example, the bis(glycol) terephthalate may be selected from the group consisting of bis(diethylene glycol) terephthalate, bis(isosorbide) terephthalate, bis(1,4-cyclohexanedimethanol) terephthalate, and derivatives thereof.

The total content of recycled monomers in the polyester resin of the present invention may be 1% by weight or more, 5% by weight or more, 10% by weight or more, 30% by weight or more, 50% by weight or more, 70% by weight or more, or 90% by weight or more. In addition, the total content of recycled monomers may be 100% by weight or less, 99% by weight or less, 80% by weight or less, 60% by weight or less, 40% by weight or less, or 20% by weight or less. As a specific example, the polyester resin may comprise the bis(2-hydroxyethyl) terephthalate and bis(glycol) terephthalate in a total amount of 30% by weight or more based on the weight of the polyester resin.

Since monomers (i.e., virgin monomers) generally used in the polymerization of a polyester resin, for example, at least one selected from the group consisting of a dicarboxylic acid, a dicarboxylic acid derivative, a diol, and a diol derivative as described above, are further used in addition to recycled monomers in the preparation of the polyester resin, the polyester resin may further comprise these comonomers.

Since bis(2-hydroxyethyl) terephthalate used in the preparation of the polyester resin of the present invention has a structure in which two ethylene glycols and one terephthalic acid are bonded, the polyester resin of the present invention may comprise a repeat unit derived from ethylene glycol and terephthalic acid. In addition, since the bis(glycol) terephthalate has a structure in which two ethylene glycols each having 3 or more carbon atoms and one terephthalic acid are bonded, the polyester resin of the present invention may further comprise a repeat unit derived from a glycol having 3 or more carbon atoms.

As described above, the polyester resin of the present invention comprises a dicarboxylic acid component and a glycol component as monomer components (polymer constituent unit) constituting the same. These may be derived from bis(2-hydroxyethyl) terephthalate and the glycol having 3 or more carbon atoms initially introduced for the preparation of the polyester resin, or a recycled bis(glycol) terephthalate prepared therefrom, and comonomers additionally introduced.

Accordingly, the polyester resin of the present invention may be a copolymerized resin comprising two or more dicarboxylic acid components and/or two or more diol components.

According to an embodiment, the diol component may further comprise a diol component, other than ethylene glycol, as a comonomer. The comonomer may comprise, for example, at least one selected from the group consisting of diethylene glycol, cyclohexanedimethanol, cyclohexanedimethanol derivatives, and isosorbide.

The diethylene glycol may contribute to enhancing the transparency and impact resistance of a polyester resin. For example, the diethylene glycol may be employed in an amount of 0.1% by mole to 50% by mole based on the number of moles of the entire diols.

The cyclohexanedimethanol (e.g., 1,2-cyclohexanedimethanol, 1,3-cyclohexanedimethanol, and 1,4-cyclohexanedimethanol) may contribute to enhancing the transparency and impact resistance of a polyester resin produced. For example, cyclohexanedimethanol may be employed in an amount of 5% by mole to 90% by mole based on the number of moles of the entire diol components. The cyclohexanedimethanol derivative may be 4-(hydroxymethyl)cyclohexylmethyl-4-(hydroxymethyl)cyclohexanecarboxylate or 4-(4-(hydroxymethyl)cyclohexylmethoxymethyl)cyclohexylmethanol. The cyclohexanedimethanol derivatives may be employed in an amount of 0.1% by mole to 25% by mole based on the number of moles of the entire diol components.

The isosorbide may enhance the processability of a final polyester resin. Although the transparency and impact resistance of a polyester resin are enhanced by the diol component of cyclohexanedimethanol and ethylene glycol, shear fluidization characteristics should be improved and the crystallization rate should be delayed for processability; however, it is difficult to achieve this effect with cyclohexanedimethanol and ethylene glycol alone. Thus, if isosorbide is employed as a diol component, the shear fluidization characteristics are improved and the crystallization rate is delayed while transparency and impact resistance are maintained, whereby it is possible to improve the processability of a polyester resin produced. Preferably, isosorbide may be employed in an amount of 0.1% by mole to 70% by mole based on the number of moles of the entire diol components.

The polyester resin may comprise terephthalic acid as a dicarboxylic acid component. For example, terephthalic acid may be employed in an amount of 5% by mole to 100% by mole based on the number of moles of the entire dicarboxylic acid. In addition, the terephthalic acid component may be formed from a terephthalic acid alkyl ester such as dimethyl terephthalic acid.

In addition, the dicarboxylic acid component may further comprise an aromatic dicarboxylic acid component, an aliphatic dicarboxylic acid component, or mixed components thereof, other than terephthalic acid. The dicarboxylic acid other than terephthalic acid may be employed in an amount of 1% by mole to 30% by mole based on the weight of the entire dicarboxylic acid components.

The aromatic dicarboxylic acid component may be an aromatic dicarboxylic acid having 8 to 20 carbon atoms, preferably, 8 to 14 carbon atoms, or a mixture thereof. Examples of the aromatic dicarboxylic acid include isophthalic acid, naphthalenedicarboxylic acids such as 2,6-naphthalenedicarboxylic acid, diphenyl dicarboxylic acid, 4,4'-stilbendicarboxylic acid, 2,5-furandicarboxylic acid, 2,5-thiophenedicarboxylic acid, and the like, but it is not limited thereto.

The aliphatic dicarboxylic acid component may be an aliphatic dicarboxylic acid having 4 to 20 carbon atoms, preferably, 4 to 12 carbon atoms, or a mixture thereof. Examples of the aliphatic dicarboxylic acid include linear, branched, or cyclic aliphatic dicarboxylic acid components such as cyclohexanedicarboxylic acids such as 1,4-cyclohexanedicarboxylic acid and 1,3-cyclohexanedicarboxylic acid, phthalic acid, sebacic acid, succinic acid, isodecylsuccinic acid, maleic acid, fumaric acid, adipic acid, glutaric acid, azelaic acid, and the like, but it is not limited thereto.

The polyester resin may also comprise a catalyst that has been used in the polymerization reaction for its preparation. For example, the polyester resin may comprise at least one catalyst selected from metal oxides and acetates. The metal contained in the catalyst may be selected from the group consisting of antimony (Sb), titanium (Ti), germanium (Ge), manganese (Mn), cobalt (Co), tin (Sn), and calcium (Ca).

The polyester resin may have a value obtained by subtracting the b value from the L value of 88 or more, 89 or more, 90 or more, 91 or more, 92 or more, or 93 or more, when Hunter Lab color space is measured. In addition, the upper limit of the L - b value is not particularly limited. But it may be, for example, 100 or less, 99 or less, 98 or less, 97 or less, or 95 or less. The measurement of the Hunter Lab color space may be carried out by making a specimen having a thickness of 6 mm with the polyester resin. As a specific example, the polyester resin may have a value obtained by subtracting the b value from the L value of 88 or more under the condition of a thickness of 6 mm when Hunter Lab color space is measured.

The intrinsic viscosity of the polyester resin at 35°C may be 0.5 dl/g or more, 0.6 dl/g or more, or 0.7 dl/g or more, and may be 1.2 dl/g or less, 1.1 dl/g or less, 1.0 dl/g or less, or 0.9 dl/g or less. For example, the polyester resin may have an intrinsic viscosity of 0.5 dl/g to 1.2 dl/g at 35°C. Specifically, the polyester resin may have an intrinsic viscosity of 0.5 dl/g to 0.9 dl/g at 35°C.

The polyester resin according to the present invention can be used as a material for beverage-filling containers, packaging films, audio and video films, and the like by virtue of its excellent color, mechanical strength, thermal resistance, transparency, and gas barrier properties. In addition, polyester sheets or plates prepared from the polyester resin according to the present invention have good transparency and excellent mechanical strength, so that they can be used as raw materials for cases, boxes, partitions, shelves, panels, packaging materials, building materials, interior and exterior materials, and the like. In addition, the polyester resin according to the present invention can be used as an industrial material such as medical fibers and tire cords.

Accordingly, the present invention provides an article, which comprises the polyester resin. As an example, the article may be a film, a sheet, or a profile. Specific examples of the film include a heat shrinkable film and a blown film. The profile refers to a continuously extrusion-molded article of plastics excluding sheets and films. It may be manufactured by a general extrusion molding method and may have, for example, a tube or channel shape.

### Mode for the Invention

Hereinafter, the present invention will be described in more detail with reference to embodiments. However, these examples are provided only for illustration purposes, and the present invention is not limited thereto.

In the glycols used in the transesterification reaction and the dicarboxylic acids or diols used as comonomers in the polymerization of a polyester resin in the following examples, unless it is indicated as a recycled monomer (e.g., terephthalic acid, ethylene glycol, or the like), it should be understood that a virgin monomer was used.

### Preparation Example 1: Recycled bis(2-hydroxyethyl) terephthalate

Recycled BHET of various purities was prepared by the depolymerization of a waste polyester resin by a known method or purchased commercially. The table below shows the peak area fraction (%) of BHET only, that is, purity, in the HPLC results of each recycled BHET measured using HPLC.

**[Table 1]**

| Recycled BHET | #1 | #2 | #3 | #4 | #5 | #6 | #7 | #8 | #9 | #3a | #4a | #7a |
|---|---|---|---|---|---|---|---|---|---|---|---|---|
| Purity (HPLC - BHET%) | 90 | 60 | 80 | 90 | 75 | 85 | 78 | 75 | 97 | 80 | 90 | 78 |

### Preparation Example 2: Preparation of a bis(glycol) terephthalate through a transesterification reaction

A 1-liter reactor for a transesterification reactor equipped with a column and a condenser that can be cooled by water was charged with 1,4-cyclohexanedimethanol as a glycol (CHDM, 641 g) and zinc acetate (0.003 part by weight relative to 100 parts by weight of the monomer mixture in the reactor) as a reaction catalyst. Then, the pressure in the reactor was adjusted to normal pressure (1.0 kg/cm²) by flowing nitrogen, and the temperature was raised while the pressure was maintained with stirring. When the temperature in the reactor reached about 200°C, while the temperature was raised to 220°C over 3 hours, bis(2-hydroxyethyl) terephthalate (BHET, 254 g) was fed to carry out a transesterification reaction. Glycols as by-products were discharged through the column and condenser during the reaction. Even upon completion of the addition of BHET, the transesterification reaction was continued while maintaining 220°C until the discharge of glycols stopped. Upon completion of the transesterification reaction, nitrogen in the pressurized reactor was released to the outside to lower the pressure in the reactor to normal pressure to obtain bis(1,4-cyclohexanedimethanol) terephthalate.

### Example 1: Preparation of a polyester resin

### Step A: Preparation of a bis(glycol) terephthalate through a transesterification reaction

Bis(2-hydroxyethyl) terephthalate (BHET #1) was subjected to a transesterification reaction with diethylene glycol (DEG), isosorbide (ISB), and 1,4-cyclohexanedimethanol (CHDM), respectively, according to the method of Preparation Example 2 above to obtain bis(diethylene glycol) terephthalate (BHDT), bis(isosorbide) terephthalate (BHIT), and bis(1,4-cyclohexanedimethinol) terephthalate (BHCT), respectively.

### Step B: Preparation of a polyester resin through a polycondensation reaction

An esterification reactor was charged with bis(2-hydroxyethyl) terephthalate (BHET #1, 3,570.2 g), bis(diethylene glycol) terephthalate (BHDT, 69.7 g), bis(isosorbide) terephthalate (BHIT, 42.2 g), bis(1,4-cyclohexanedimethinol) terephthalate (BHCT, 212.9 g), terephthalic acid (TPA, 913.0 g), ethylene glycol (EG, 46.3 g), isosorbide (ISB, 9.9 g), a Ge catalyst (2.6 g), a blue toner (0.012 g), and a red toner (0.006 g).

Subsequently, nitrogen was injected into the esterification reactor to make the reactor pressurized by 2.0 kgf/cm² higher than normal pressure (absolute pressure: 2,231.1 mmHg). Then, the temperature of the esterification reactor was raised to 220°C over 90 minutes and maintained at 220°C for 2 hours, and the temperature was then raised again to 260°C over 2 hours. The mixture in the esterification reactor was stored at 260°C for about 7 hours and then transferred to a polycondensation reactor, and by-products formed during the reaction were discharged through a column and a condenser.

Then, the pressure of the polycondensation reactor was reduced from normal pressure to 5 Torr (absolute pressure: 5 mmHg) over 30 minutes. At the same time, the temperature of the polycondensation reactor was raised to 280°C over 1 hour, and a polycondensation reaction was then carried out while the pressure of the polycondensation reactor was maintained at 1 Torr (absolute pressure: 1 mmHg) or less. At the beginning of the polycondensation reaction, the stirring speed was set high. As the polycondensation reaction proceeded, when the stirring power was weakened due to the increase in the viscosity of the reactants or the temperature of the reactants rose above the set temperature, the stirring speed was appropriately adjusted accordingly. The polycondensation reaction was carried out until the intrinsic viscosity (IV) of the mixture (melt) in the reactor reached 0.60 dl/g. Thereafter, the mixture was discharged to the outside of the reactor to form pellets, which were solidified with a cooling liquid and then granulated to have an average weight of about 12 to 14 mg to prepare about 4 kg of a polyester resin (copolymer).

### Example 2: Preparation of a polyester resin

The same procedure as in Example 1 was repeated to obtain polyester granules, except that, in step A, bis(2-hydroxyethyl) terephthalate (BHET #2) was subjected to a transesterification reaction with diethylene glycol (DEG) to obtain bis(diethylene glycol) terephthalate (BHDT); and that, in step B, the reactor was charged with bis(2-hydroxyethyl) terephthalate (BHET #2, 2,594.0 g), bis(diethylene glycol) terephthalate (BHDT, 174.6 g), terephthalic acid (TPA, 1,610.5 g), ethylene glycol (EG, 671.2 g), 1,4-cyclohexanedimethanol (CHDM, 58.8 g), a Ge catalyst (2.6 g), a Ti catalyst (0.4 g), phosphoric acid (0.4 g), a blue toner (0.016 g), and a red toner (0.004 g), the esterification reaction was carried out at a pressure higher than normal pressure by 0.5 kgf/cm² and a temperature of 260°C, and the polycondensation reaction was carried out at a temperature of 275°C until the intrinsic viscosity (IV) reached 0.70 dl/g. The granules were left at 150°C for 1 hour to be crystallized, which were then fed to a solid-state polymerization reactor. While nitrogen flowed at a rate of 50 L/minute, the temperature of the reactor was raised from room temperature to 200°C at a rate of 40°C/hour. While it was maintained, a solid-state polymerization was carried out until the intrinsic viscosity (IV) of the granules in the reactor reached 0.90 dl/g to obtain about 4 kg of a polyester resin (copolymer).

### Example 3: Preparation of a polyester resin

The same procedure as in Example 1 was repeated to obtain about 4 kg of a polyester resin (copolymer), except that, in step A, bis(2-hydroxyethyl) terephthalate (BHET #3) was subjected to a transesterification reaction with 1,4-cyclohexanedimethanol (CHDM) to obtain bis(1,4-cyclohexanedimethanol) terephthalate (BHCT); and that, in step B, the reactor was charged with bis(2-hydroxyethyl) terephthalate (BHET #3, 3,941.2 g), bis(1,4-cyclohexanedimethanol) terephthalate (BHCT, 1,144.6 g), ethylene glycol (EG, 34.0 g), 1,4-cyclohexanedimethanol (CHDM, 52.6 g), diethylene glycol (DEG, 38.7 g), a CHDM derivative (comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3, 80.0 kg), a Ge catalyst (5.1 g), a Ti catalyst (0.4 g), phosphoric acid (0.4 g), a blue toner (0.010 g), and a red toner (0.002 g), the esterification reaction was carried out at a pressure higher than normal pressure by 2.0 kgf/cm² and a temperature of 255°C, and the polycondensation reaction was carried out at a temperature of 285°C until the intrinsic viscosity (IV) reached 0.78 dl/g.

### Example 4: Preparation of a polyester resin

The same procedure as in Example 1 was repeated to obtain about 4 kg of a polyester resin (copolymer), except that, in step A, bis(2-hydroxyethyl) terephthalate (BHET #4) was subjected to a transesterification reaction with 1,4-cyclohexanedimethanol (CHDM) to obtain bis(1,4-cyclohexanedimethanol) terephthalate (BHCT); and that, in step B, the reactor was charged with bis(2-hydroxyethyl) terephthalate (BHET #4, 2,782.0 g), bis(1,4-cyclohexanedimethanol) terephthalate (BHCT, 763.1 g), terephthalic acid (TPA, 909.1 g), ethylene glycol (EG, 147.1 g), 1,4-cyclohexanedimethanol (CHDM, 315.4 g), diethylene glycol (DEG, 38.7 g), a CHDM derivative (comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3, 80.0 kg), a Ge catalyst (5.1 g), a Ti catalyst (0.4 g), phosphoric acid (0.4 g), cobalt acetate (0.5 g), a blue toner (0.002 g), and a red toner (0.001 g), the esterification reaction was carried out at a pressure higher than normal pressure by 2.0 kgf/cm² and a temperature of 255°C, and the polycondensation reaction was carried out at a temperature of 285°C until the intrinsic viscosity (IV) reached 0.78 dl/g.

### Example 5: Preparation of a polyester resin

The same procedure as in Example 1 was repeated to obtain about 4 kg of a polyester resin (copolymer), except that, in step A, bis(2-hydroxyethyl) terephthalate (BHET #5) was subjected to a transesterification reaction with 1,4-cyclohexanedimethanol (CHDM) to obtain bis(1,4-cyclohexanedimethanol) terephthalate (BHCT); and that, in step B, the reactor was charged with bis(2-hydroxyethyl) terephthalate (BHET #5, 907.1 g), bis(1,4-cyclohexanedimethanol) terephthalate (BHCT, 2,596.3 g), terephthalic acid (TPA, 953.7 g), ethylene glycol (EG, 134.8 g), a Ti catalyst (0.1 g), phosphoric acid (0.8 g), a blue toner (0.002 g), and a red toner (0.001 g), the esterification reaction was carried out at a pressure higher than normal pressure by 1.5 kgf/cm² and a temperature of 250°C, and the polycondensation reaction was carried out at a temperature of 270°C until the intrinsic viscosity (IV) reached 0.82 dl/g.

### Example 6: Preparation of a polyester resin

The same procedure as in Example 1 was repeated to obtain about 4 kg of a polyester resin (copolymer), except that, in step A, bis(2-hydroxyethyl) terephthalate (BHET #6) was subjected to a transesterification reaction with diethylene glycol (DEG) and 1,4-cyclohexanedimethanol (CHDM) to obtain bis(diethylene glycol) terephthalate (BHDT) and bis(1,4-cyclohexanedimethanol) terephthalate (BHCT); and that, in step B, the reactor was charged with bis(2-hydroxyethyl) terephthalate (BHET #6, 1,376.8 g), bis(diethylene glycol) terephthalate (BHDT, 278.1 g), bis(1,4-cyclohexanedimethanol) terephthalate (BHCT, 1,132.9 g), terephthalic acid (TPA, 1,514.7 g), ethylene glycol (EG, 784.2 g), 1,4-cyclohexanedimethanol (CHDM, 26.0 g), a Ti catalyst (0.1 g), phosphoric acid (0.8 g), cobalt acetate (1.1 g), a blue toner (0.002 g), and a red toner (0.001 g), the esterification reaction was carried out at a pressure higher than normal pressure by 1.5 kgf/cm² and a temperature of 250°C, and the polycondensation reaction was carried out at a temperature of 270°C until the intrinsic viscosity (IV) reached 0.82 dl/g.

### Example 7: Preparation of a polyester resin

The same procedure as in Example 1 was repeated to obtain about 4 kg of a polyester resin (copolymer), except that, in step A, bis(2-hydroxyethyl) terephthalate (BHET #7) was subjected to a transesterification reaction with isosorbide (ISB) and 1,4-cyclohexanedimethanol (CHDM) to obtain bis(isosorbide) terephthalate (BHIT) and bis(1,4-cyclohexanedimethanol) terephthalate (BHCT); and that, in step B, the reactor was charged with bis(2-hydroxyethyl) terephthalate (BHET #7, 1,072.3 g), bis(isosorbide) terephthalate (BHIT, 629.2 g), bis(1,4-cyclohexanedimethanol) terephthalate (BHCT, 2,258.7 g), terephthalic acid (TPA, 953.1 g), ethylene glycol (EG, 83.8 g), 1,4-cyclohexanedimethanol (CHDM, 24.3 g), recycled isosorbide (r-ISB, 49.3 g), diethylene glycol (DEG, 35.8 g), a Ge catalyst (25.6 g), phosphoric acid (0.08 g), a blue toner (0.012 g), and a red toner (0.004 g), the esterification reaction was carried out at a pressure higher than normal pressure by 1.0 kgf/cm² and a temperature of 265°C, and the polycondensation reaction was carried out at a temperature of 275°C until the intrinsic viscosity (IV) reached 0.70 dl/g.

### Example 8: Preparation of a polyester resin

The same procedure as in Example 1 was repeated to obtain about 4 kg of a polyester resin (copolymer), except that, in step A, bis(2-hydroxyethyl) terephthalate (BHET #8) was subjected to a transesterification reaction with diethylene glycol (DEG) and 1,4-cyclohexanedimethanol (CHDM) to obtain bis(diethylene glycol) terephthalate (BHDT) and bis(1,4-cyclohexanedimethanol) terephthalate (BHCT); and that, in step B, the reactor was charged with bis(2-hydroxyethyl) terephthalate (BHET #8, 483.4 g), bis(diethylene glycol) terephthalate (BHDT, 358.0 g), bis(1,4-cyclohexanedimethanol) terephthalate (BHCT, 556.9 g), terephthalic acid (TPA, 2,448.4 g), recycled ethylene glycol (r-EG, 684.4 g), recycled 1,4-cyclohexanedimethanol (r-CHDM, 13.7 g), diethylene glycol (DEG, 10.1 g), a CHDM derivative (comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3, 166.7 kg), a Ge catalyst (2.6 g), phosphoric acid (0.4 g), a blue toner (0.020 g), and a red toner (0.008 g), the esterification reaction was carried out at a pressure higher than normal pressure by 0.5 kgf/cm² and a temperature of 260°C, and the polycondensation reaction was carried out at a temperature of 275°C until the intrinsic viscosity (IV) reached 0.75 dl/g.

### Example 9: Preparation of a polyester resin

The same procedure as in Example 1 was repeated to obtain polyester granules, except that, in step A, bis(2-hydroxyethyl) terephthalate (BHET #9) was subjected to a transesterification reaction with 1,4-cyclohexanedimethanol (CHDM) to obtain bis(1,4-cyclohexanedimethanol) terephthalate (BHCT); and that, in step B, the reactor was charged with bis(2-hydroxyethyl) terephthalate (BHET #9, 3,621.9 g), bis(1,4-cyclohexanedimethanol) terephthalate (BHCT, 212.9 g), recycled terephthalic acid (r-TPA, 929.9 g), isophthalic acid (IPA, 2,451.6 g), ethylene glycol (EG, 21.0 g), isosorbide (ISB, 39.7 g), recycled diethylene glycol (r-DEG, 21.6 g), diethylene glycol (DEG, 21.6 g), a Ti catalyst (0.4 g), phosphoric acid (8.0 g), cobalt acetate (0.7 g), a blue toner (0.020 g), and a red toner (0.008 g), the esterification reaction was carried out at a pressure higher than normal pressure by 3.0 kgf/cm² and a temperature of 280°C, and the polycondensation reaction was carried out at a temperature of 280°C until the intrinsic viscosity (IV) reached 0.60 dl/g. The granules were left at 150°C for 1 hour to be crystallized, which were then fed to a solid-state polymerization reactor. While nitrogen flowed at a rate of 50 L/minute, the temperature of the reactor was raised from room temperature to 190°C at a rate of 40°C/hour. While it was maintained, a solid-state polymerization was carried out until the intrinsic viscosity (IV) of the granules in the reactor reached 1.10 dl/g to obtain about 4 kg of a polyester resin (copolymer).

### Comparative Example 1: Preparation of a polyester resin

The same procedure as in Example 3 was repeated to obtain about 4 kg of a polyester resin (copolymer), except that step A was not carried out; and that, in step B, the reactor was charged with bis(2-hydroxyethyl) terephthalate (BHET #3a, 4,636.7 g), ethylene glycol (EG, 56.6 g), 1,4-cyclohexanedimethanol (CHDM, 841.2 g), a CHDM derivative (comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3, 80.0 kg), a Ge catalyst (5.1 g), a Ti catalyst (0.4 g), phosphoric acid (0.4 g), a blue toner (0.010 g), and a red toner (0.002 g), the esterification reaction was carried out at a pressure higher than normal pressure by 2.0 kgf/cm² and a temperature of 255°C, and the polycondensation reaction was carried out at a temperature of 285°C until the intrinsic viscosity (IV) reached 0.78 dl/g.

### Comparative Example 2: Preparation of a polyester resin

The same procedure as in Example 1 was repeated to obtain about 4 kg of a polyester resin (copolymer), except that step A was not carried out; and that, in step B, the reactor was charged with bis(2-hydroxyethyl) terephthalate (BHET #4a, 3,709.4 g), terephthalic acid (TPA, 606.1 g), ethylene glycol (EG, 56.6 g), 1,4-cyclohexanedimethanol (CHDM, 841.2 g), a CHDM derivative (comprising 4-(hydroxymethyl)cyclohexylmethyl-4-(hydroxymethyl)cyclohexanecarboxylate and 4-(4-(hydroxymethyl)cyclohexylmethoxymethyl)cyclohexylmethanol at a molar ratio of 1:3, 80.0 kg), a Ge catalyst (5.1 g), a Ti catalyst (0.4 g), phosphoric acid (0.4 g), cobalt acetate (0.5 g), a blue toner (0.002 g), and a red toner (0.001 g), the esterification reaction was carried out at a pressure higher than normal pressure by 2.0 kgf/cm² and a temperature of 255°C, and the polycondensation reaction was carried out at a temperature of 285°C until the intrinsic viscosity (IV) reached 0.80 dl/g.

### Comparative Example 3: Preparation of a polyester resin

The same procedure as in Example 1 was repeated to obtain about 4 kg of a polyester resin (copolymer), except that step A was not carried out; and that, in step B, the reactor was charged with bis(2-hydroxyethyl) terephthalate (BHET #7a, 1,072.3 g), terephthalic acid (TPA, 2,102.3 g), ethylene glycol (EG, 83.8 g), 1,4-cyclohexanedimethanol (CHDM, 1,580.5 g), recycled isosorbide (r-ISB, 493.1 g), diethylene glycol (DEG, 35.8 g), a Ge catalyst (25.6 g), phosphoric acid (0.08 g), a blue toner (0.012 g), and a red toner (0.004 g), the esterification reaction was carried out at a pressure higher than normal pressure by 1.0 kgf/cm² and a temperature of 265°C, and the polycondensation reaction was carried out at a temperature of 275°C until the intrinsic viscosity (IV) reached 0.70 dl/g.

The monomer compositions of the Examples and Comparative Examples are summarized in Tables 2 and 3 below.

**[Table 2]**

| | | Ex. 1 | Ex. 2 | Ex. 3 | Ex. 4 | Ex. 5 | Ex. 6 |
|---|---|---|---|---|---|---|---|
| Recycled monomers in the resin (% by weight) | | 80 | 54 | 96 | 70 | 76 | 55 |
| Virgin monomer (mole) | TPA | 5.50 | 9.70 | 0.00 | 5.48 | 5.75 | 9.12 |
| | EG | 0.75 | 10.83 | 0.55 | 2.37 | 2.17 | 12.65 |
| | DEG | 0.00 | 0.00 | 0.37 | 0.37 | 0.00 | 0.00 |
| | CHDM | 0.00 | 0.41 | 0.37 | 2.19 | 0.00 | 0.18 |
| | CHDM derivative | 0.00 | 0.00 | 0.30 | 0.30 | 0.00 | 0.00 |
| | ISB | 0.07 | 0.00 | 0.00 | 0.00 | 0.00 | 0.00 |
| Recycled BHET No. | | #1 | #2 | #3 | #4 | #5 | #6 |
| Recycled monomer | BHET | 14.06 | 10.21 | 15.52 | 10.95 | 3.57 | 5.42 |
| | BHDT | 0.20 | 0.51 | 0.00 | 0.00 | 0.00 | 0.81 |
| (mole) | BHIT | 0.10 | 0.00 | 0.00 | 0.00 | 0.00 | 0.00 |
| | BHCT | 0.51 | 0.00 | 2.74 | 1.83 | 6.21 | 2.71 |
| * The amount (mole) of each virgin monomer and recycled monomer added refers to the relative molar ratio. | | | | | | | |

**[Table 3]**

| | | Ex. 7 | Ex. 8 | Ex. 9 | C. Ex. 1 | C. Ex. 2 | C. Ex. 3 |
|---|---|---|---|---|---|---|---|
| Recycled monomers in the resin (% by weight) | | 79 | 35 | 60 | 83 | 70 | 22 |
| Virgin monomer (mole) | TPA | 5.74 | 14.75 | 0.00 | 0.00 | 3.65 | 12.66 |
| | EG | 1.35 | 0.00 | 0.34 | 0.91 | 0.91 | 1.35 |
| | DEG | 0.34 | 0.10 | 0.21 | 0.00 | 0.00 | 0.34 |
| | CHDM | 0.17 | 0.00 | 0.00 | 5.84 | 5.84 | 10.98 |
| | CHDM derivative | 0.00 | 0.62 | 0.00 | 0.30 | 0.30 | 0.00 |
| | ISB | 0.00 | 0.00 | 0.27 | 0.00 | 0.00 | 0.00 |
| Recycled BHET No. | | #7 | #8 | #9 | #3a | #4a | #7a |
| Recycled monomer (mole) | BHET | 4.22 | 1.90 | 14.26 | 18.25 | 14.60 | 4.22 |
| | BHDT | 0.00 | 1.05 | 0.00 | 0.00 | 0.00 | 0.00 |
| | BHIT | 1.52 | 0.00 | 0.00 | 0.00 | 0.00 | 0.00 |
| | BHCT | 5.40 | 1.33 | 0.51 | 0.00 | 0.00 | 0.00 |
| | r-TPA | 0.00 | 0.00 | 5.60 | 0.00 | 0.00 | 0.00 |
| | r-EG | 0.00 | 11.04 | 0.00 | 0.00 | 0.00 | 0.00 |
| | r-DEG | 0.00 | 0.00 | 0.21 | 0.00 | 0.00 | 0.00 |
| | r-CHDM | 0.00 | 0.10 | 0.00 | 0.00 | 0.00 | 0.00 |
| | r-ISB | 0.34 | 0.00 | 0.00 | 0.00 | 0.00 | 3.38 |
| * The amount (mole) of each virgin monomer and recycled monomer added refers to the relative molar ratio. | | | | | | | |

### Test Example

The raw materials used and final resins in the Examples and Comparative Examples were tested as follows.

### (1) Amount of recycled BHET added

Moles of bis(2-hydroxyethyl) terephthalate (BHET) added based on the total number of moles of dicarboxylic acid and its derivatives (TPA, BHET, BHDT, BHIT, and BHCT) added for the preparation of a polyester resin was calculated in percentage (% by mole).

### (2) Purity of recycled BHET (HPLC)

About 0.01 g of recycled bis(2-hydroxyethyl) terephthalate was diluted in about 20 ml of methanol, which was analyzed by high-performance liquid chromatography (HPLC) (model: Waters e2695, column: C18 (4.6 × 250 mm), 5 µm, UV detector: 242 nm, injection volume: 10 µl, eluent (gradient) A: H₂O + H₃PO₄, B: acetonitrile) Thereafter, the fraction (%) of only the BHET peak area among the total peak area of HPLC was obtained.

### (3) Content of the recycled monomers in the resin

The total weight of only the recycled monomers (BHET, BHDT, BHIT, BHCT, r-EG, r-CHDM, r-DEG, r-TPA, and r-IPA) in percentage (% by weight) was calculated based on the total weight of the monomers added for the preparation of the polyester resin (BHET, BHDT, BHIT, BHCT, TPA, IPA, EG, CHDM, ISB, DEG, r-EG, r-CHDM, r-DEG, r-TPA, r-IPA, and the like).

### (4) Copolymerization composition (NMR)

Each polyester resin was dissolved in a CDCl₃ solvent at a concentration of 3 mg/ml, whose ¹H-NMR spectrum was obtained at 25°C using nuclear magnetic resonance equipment (JEOL, 600MHz FT-NMR). The content (% by mole) of residues derived from diethylene glycol (DEG), isosorbide (ISB), cyclohexanedimethanol (CHDM), and derivatives thereof, based on the total number of moles of residues derived from all glycols (EG, DEG, ISB, CHDM, and the like) was calculated by analyzing the above spectra, respectively.

As a result, it was confirmed that the copolymerization compositions of the polyester resins of the Examples and Comparative Examples were diverse. Specifically, it was confirmed that the contents of residues derived from glycols (DEG, ISB, CHDM, and the like) and derivatives thereof in each polyester resin were diverse.

However, some of the polyester resins had the same composition. Specifically, the copolymer compositions of Comparative Example 1 and Example 3, Comparative Example 2 and Example 4, and Comparative Example 3 and Example 7 were confirmed to be the same.

### (5) Color (Hunter Lab)

The chromaticity and brightness of the polyester resins were measured using a Varian Cary 5 UV/Vis/NIR spectrophotometer equipped with a diffuse reflection accessory. A polyester resin specimen with a thickness of 6 mm was prepared through injection molding at 250°C, for which transmittance data with Illuminant D65 at an observer's angle of 2° were obtained and processed using a color analyzer in Grams/32 software to calculate Hunter Lab values. The values of subtracting the b value from the L value (L - b) were calculated.

The test results are shown in Tables 4 and 5 below.

**[Table 4]**

| | | Unit | Ex. 1 | Ex. 2 | Ex. 3 | Ex. 4 | Ex. 5 | Ex. 6 |
|---|---|---|---|---|---|---|---|---|
| Recycled BHET | Added amount | % by mole | 69 | 50 | 85 | 60 | 23 | 30 |
| | Purity | % | 90 | 60 | 80 | 90 | 75 | 85 |
| Recycled monomers in the resin | | % by weight | 80 | 54 | 96 | 70 | 76 | 55 |
| Resin color (6T col L-b) | | | 92 | 93 | 89 | 88 | 89 | 91 |

**[Table 5]**

| | | Unit | Ex. 7 | Ex. 8 | Ex. 9 | C. Ex. 1 | C. Ex. 2 | C. Ex. 3 |
|---|---|---|---|---|---|---|---|---|
| Recycled BHET | Added amount | % by mole | 25 | 9 | 70 | 100 | 80 | 25 |
| | Purity | % | 78 | 75 | 97 | 80 | 90 | 78 |
| Recycled monomers in the resin | | % by weight | 79 | 35 | 60 | 83 | 70 | 22 |
| Resin color (6T col L-b) | | | 88 | 88 | 91 | 87 | 88 | 88 |

As can be seen from Tables 4 and 5 above, in Examples 1 to 9, in which some of BHET as a recycled monomer was transesterified with other glycols (DEG, ISB, CHDM) to obtain BHDT, BHIT, and BHCT, which were then added to the polymerization reaction to obtain a polyester resin, the color quality of the polyester resins was excellent while the total content of recycled monomers was increased. In particular, in Examples 7 to 9, additional recycled glycols (r-EG, r-DEG, r-CHDM, or r-ISB) and recycled carboxylic acid (r-TPA) were further used to increase the total content of recycled monomers.

In contrast, in Comparative Example 1, which had the same copolymerization composition as that of Example 3, BHET alone was used as a recycled monomer, which lowered the content of recycled monomers in the resin, and the color of the final polyester resin was poor due to the low purity of BHET. In addition, in Comparative Example 2 in which BHET alone was used as a recycled monomer, enhancements in purity through transesterification with a glycol were not achieved; thus, the color of the final polyester resin was poor as compared with Example 1 in which BHET with the same purity was used. In addition, in Comparative Example 3, which had almost the same amounts of BHET and r-ISB as those of Example 3, other recycled monomers were not used, which lowered the total content of recycled monomers, and the color of the final polyester resin was poor.

## Claims

1. A process for preparing a polyester resin, which comprises:
subjecting bis(2-hydroxyethyl) terephthalate to a reaction with a glycol having 3 or more carbon atoms to prepare a bis(glycol) terephthalate; and
preparing a copolymer using the bis(glycol) terephthalate and bis(2-hydroxyethyl) terephthalate.

2. The process for preparing a polyester resin of claim 1, wherein the bis(2-hydroxyethyl) terephthalate is obtained by the depolymerization of waste polyester.

3. The process for preparing a polyester resin of claim 1, wherein the step of preparing the bis(glycol) terephthalate comprises:
feeding a glycol having 3 or more carbon atoms to a reactor; and
feeding bis(2-hydroxyethyl) terephthalate to the reactor and carrying out a transesterification reaction.

4. The process for preparing a polyester resin of claim 1, wherein the glycol having 3 or more carbon atoms comprises at least one selected from the group consisting of 1,3-propanediol, 1,4-butanediol, 1,4-cyclohexanedimethanol, isosorbide, 2-methyl-1,3-propanediol, 2-methylene-1,3-propanediol, 2-ethyl-1,3-propanediol, 2-isopropyl-1,3-propanediol, 2,2-dimethyl-1,3-propanediol, 2,3-butanediol, 3-methyl-1,5-pentanediol, 3-methyl-2,4-pentanediol, 1,6-hexanediol, 1,2-cyclohexanediol, 1,4-cyclohexanediol, diethylene glycol, polyethylene glycol, polypropylene glycol, polytetramethylene glycol, polyhexamethylene glycol, copolymers of ethylene oxide and tetrahydrofuran, ethylene oxide-adducted polypropylene glycol, polycarbonate diol, polyneopentyl glycol, poly-3-methylpentanediol, poly-1,5-pentanediol, and derivatives thereof.

5. The process for preparing a polyester resin of claim 1, wherein the glycol having 3 or more carbon atoms comprises at least one type of recycled glycol obtained by the depolymerization of waste polyester.

6. The process for preparing a polyester resin of claim 5, wherein the recycled glycol is selected from the group consisting of recycled 1,4-cyclohexanedimethanol, recycled diethylene glycol, and recycled isosorbide.

7. The process for preparing a polyester resin of claim 1, wherein the bis(2-hydroxyethyl) terephthalate has a purity of 60% to 97%.

8. The process for preparing a polyester resin of claim 1, wherein the bis(glycol) terephthalate has a purity of 80% or more.

9. The process for preparing a polyester resin of claim 1, wherein the step of preparing the copolymer comprises:
subjecting comonomers comprising the bis(glycol) terephthalate and bis(2-hydroxyethyl) terephthalate to an esterification reaction to obtain an oligomer; and
subjecting the oligomer to a polycondensation reaction to obtain the copolymer.

10. The process for preparing a polyester resin of claim 5, wherein at least one comonomer selected from the group consisting of a dicarboxylic acid, a dicarboxylic acid derivative, a diol, and a diol derivative is further introduced into the esterification reaction.

11. The process for preparing a polyester resin of claim 10, wherein the dicarboxylic acid comprises terephthalic acid or isophthalic acid;
the dicarboxylic acid derivative comprises dimethyl terephthalate or dimethyl isophthalate;
the glycol comprises diethylene glycol, 1,4-cyclohexanedimethanol, isosorbide, 1,2-propanediol, 1,3-propanediol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 1,4-butanediol, 2,3-butanediol, 3-methyl-1,5-pentanediol, 3-methyl-2,4-pentanediol, 1,6-hexanediol, 1,2-cyclohexanediol, or 1,4-cyclohexanediol; and
the diol derivative comprises 4-(hydroxymethyl)cyclohexylmethyl-4-(hydroxymethyl)cyclohexanecarboxylate or 4-(4-(hydroxymethyl)cyclohexylmethoxymethyl)cyclohexylmethanol.

12. The process for preparing a polyester resin of claim 10, wherein the at least one comonomer comprises a recycled monomer obtained by the depolymerization of waste polyester.

13. The process for preparing a polyester resin of claim 12, wherein the recycled monomer is selected from the group consisting of recycled ethylene glycol, recycled 1,4-cyclohexanedimethanol, recycled diethylene glycol, recycled isosorbide, recycled terephthalic acid, recycled dimethyl terephthalate, recycled isophthalic acid, and recycled dimethyl isophthalate.

14. A polyester resin, which comprises bis(2-hydroxyethyl) terephthalate and a bis(glycol) terephthalate as comonomers, wherein the glycol has 3 or more carbon atoms.

15. The polyester resin of claim 14, wherein the polyester resin comprises the bis(2-hydroxyethyl) terephthalate and bis(glycol) terephthalate in a total amount of 30% by weight or more based on the weight of the polyester resin.

16. The polyester resin of claim 14, wherein the polyester resin has a value obtained by subtracting the b value from the L value of 88 or more under the condition of a thickness of 6 mm when Hunter Lab color space is measured.

17. The polyester resin of claim 14, wherein the bis(glycol) terephthalate is a transesterification product between bis(2-hydroxyethyl) terephthalate and a glycol having 3 or more carbon atoms.
